# EUROPEAN PATENT SPECIFICATION

(11) **EP 3 331 365 B1**
(45) Date of publication and mention of the grant of the patent: **27.05.2020**
(21) Application number: 16747535.9
(22) Date of filing: 04.08.2016
(51) Int. Cl.: A01N 47/30, A61K 31/17, A01P 7/00

(54) **AGENT FOR FIGHTING FISH PARASITES**
MITTEL ZUR BEKÄMPFUNG VON FISCHPARASITEN
AGENT POUR LE LUTTE CONTRE LES PARASITES DE POISSONS

(30) Priority: 05.08.2015 GB 201513872
(43) Date of publication of application: 13.06.2018
(73) Proprietor: Pharmaq AS, 7863 Overhalla (NO)
(72) Inventor: WIIK-NILSEN, Christer Røss, N-0213 Oslo (NO); ANDERSEN, Rachmilla Souhoka, N-0213 Oslo (NO); KARLSEN, Marius, N-0213 Oslo (NO)
(74) Representative: Mannion, Sally Kim
(86) International application number: PCT/EP2016/068665
(87) International publication number: WO 2017/021498

(56) References cited:
- EP-A2- 0 407 343
- .: "Chapter 57.6.3.1 Pesticides Affecting Oxidative Phosphorylation - Diafenthiuron" In: "Handbook of Pesticide Toxicology Second Edition - Volume 2 - Agents", 1 January 2001 (2001-01-01), Academic Press, San Diego, U.S.A., XP055300151, ISBN: 978-0-12-426262-1 pages 1224-1225, paragraph 57.6.3.1 table 57.4
- .: "MATERIAL SAFETY DATA SHEET: Diafenthiuron", , 24 August 2010 (2010-08-24), pages 1-4, XP055300168, Shanghai, China Retrieved from the Internet: URL:http://www.spechemicals.com/uploadFile /201082451170109.pdf [retrieved on 2016-09-06]
- GRAVE ET AL: "Consumption of Drugs for sea lice Infestations in Norwegian Fish Farms: methods for assessment of treatment rates and treatment patterns", DISEASES OF AQUATIC ORGANISMS, vol. 60, 1 January 2004 (2004-01-01), pages 123-131, XP55253900,
- BURRIDGE L ET AL: "Chemical use in salmon aquaculture: A review of current practices and possible environmental effects", AQUACULTURE, ELSEVIER, AMSTERDAM, NL, vol. 306, no. 1-4, 15 August 2010 (2010-08-15), pages 7-23, XP027156509, ISSN: 0044-8486 [retrieved on 2010-05-25]

## Description

### Field of the Invention

In the broadest aspect, the present invention relates to the field of treating parasitic sea lice infestations in fish.

### Background

Parasitic infestations constitute considerable problems in the fish farming industry. Infestation with sea lice in particular (such as *Lepeophtheirus salmonis*, *Caligus elongatus,* and *Caligus rogercresseyi*) is considered to be one of the most important disease problems in the farming of salmonids, especially in Atlantic salmon (*Salmo salar*) and rainbow trout (*Oncorhynchus mykiss*)*.*

Fish parasites may feed on the host fish and can be harmful or fatal to the host. For example, sea lice infestation can cause fin damage, skin erosion, bleeding, and open wounds leading to infection with other pathogens. More damage may be caused when infected fish jump or scrape along nets in an attempt to dislodge irritating parasites such as sea lice. Fish infested with parasites such as sea lice may also have reduced appetite, which is likely to have a direct effect on fish growth rates, and is of particular concern to fish farmers.

It is very difficult to prevent regular infestations of fish parasites. For example, it is difficult to control sea lice in sea water pens because sea lice are ubiquitous and transported with currents.

The control of fish parasites, such as sea lice, in fish farms is currently achieved by the periodic treatment of infected fish with various parasiticides, including avermectins (such as emamectin benzoate and ivermectin), organophosphates (such as azamethiphos and dichlorvos), pyrethroids (such as deltamethrin and cypermethrin), insect growth regulators (IGRs) (including benzoylureas such as diflubenzuron and teflubenzuron), pyrethrins, and oxidising agents (in particular, hydrogen peroxide). Examples of such treatments are described in K. Grave et al., Diseases of Aquatic Organisms 2004, 60, 123-131, L. Burridge et al., Aquaculture 2010, 306, 7-23 or EP0407343A2.

These parasiticides are normally administered as bath treatment (especially in the case of hydrogen peroxide, pyrethroids, and organophosphates) or as a feed additive (avermectins and benzoylureas). Parasiticides may also be administered to fish by intraperitoneal injection.

As a result of frequent and sub-optimal use of these parasiticides, resistance can develop. Parasites such as sea lice with reduced sensitivity to parasiticides present a big threat to the fish industry. The emergence of resistant parasites such as sea lice also encourages the use of higher doses of the parasiticides which increases costs and may be detrimental to the environment.

Moreover, where there is evidence of resistance to a particular treatment, care should be taken to avoid the use of related compounds. Hence, it is advantageous for as many different groups of effective compounds as possible to be available for use in treating infestations of fish parasites such as sea lice.

New agents capable of treating parasitic infestations in fish, such as sea lice infestations, that are safe to fish, farm workers, consumers, and the environment, are therefore required.

The thiourea diafenthiuron is an insecticide and acaricide, and it is an inhibitor of mitochondrial ATP synthase. Diafenthiuron has previously been described as being toxic to fish. There is a strong prejudice in the art that even when used in very low concentrations, diafenthiuron is harmful to fish, and LC₅₀ (96h) values for Rainbow trout of as low as 0.0007mg/l have previously been reported. Diafenthiuron has not previously been investigated or reported for use in treating fish parasites, such as sea lice.

It has surprisingly now been found, however, that diafenthiuron is highly effective at treating parasitic sea lice and is not toxic to fish such as salmon and tilapia.

### Summary

In accordance with a first aspect of the present invention, there is provided diafenthiuron, or a veterinarily acceptable salt thereof, for use in treating parasitic sea lice infestations in fish.

The parasitic sea lice may be of the Caligidae family. In particular, the sea lice may be of the *Dissonus, Lepeophtheirus,* or *Caligus* genus. The sea lice may be copepodites, pre-adult, or adult sea lice.

The fish may be of the Salmonidae family. In particular, the fish may be of the *Salmo* or *Oncorhynchus* genus. Alternatively, the fish may be of the Cichlidae family, for example, of the Oreochromis genus.

A parasitically effective amount of the diafenthiuron or diafenthiuron salt may be present in a veterinarily acceptable formulation.

The formulation may comprise a hydrophilic polymer. The hydrophilic polymer may comprise poly(ethylene glycol) or poly(propylene) glycol, or a derivative thereof.

The formulation may comprise a non-aqueous polar solvent. The non-aqueous polar solvent may comprise one or a combination of DMSO, NMP, Tetraglycol, acetone, or DMF.

The formulation may comprise a solubilizer. The solubilizer may comprise one or a combination of Cremophor EL, Tween, Brij C₁₀, Kolliphor HS₁₅, or Cremophor RH₄₀.

The formulation may be suitable for dispersing the diafenthiuron or diafenthiuron salt in water containing the fish to a final concentration of 0.05-5 ppm, preferably 0.5-2 ppm.

The diafenthiuron or diafenthiuron salt may be in the form of medicated fish feed. The medicated fish feed may be in the form of fish feed granules or pellets which have a coating comprising the diafenthiuron or diafenthiuron salt.

The diafenthiuron or diafenthiuron salt may be formulated for intraperitoneal injection. The formulation for intraperitoneal injection may comprise diafenthiuron in an amount of 10-100 mg/ml.

The diafenthiuron or diafenthiuron salt may be formulated in combination with an additional active agent. The additional active agent may be an agent that is capable of treating parasitic sea lice in fish.

Also disclosed herein is a method of treating fish parasites. The method comprises administering diafenthiuron, or a veterinarily acceptable salt thereof, to the fish. The fish parasites are sea lice.

Further disclosed herein is a composition comprising diafenthiuron, or a veterinarily acceptable salt thereof, and fish feed.

Also disclosed herein is a use of diafenthiuron, or a veterinarily acceptable salt thereof, in the treatment of sea lice in fish. The use may be substantially as described herein.

### Detailed Description

The use of diafenthiuron, or a veterinarily acceptable salt thereof, for treating parasitic sea lice in fish has not previously been described.

The terms "treating fish parasites", "treating parasites in fish", "treating parasitic infestation of fish", "treatment of parasitic infestations of fish", "treatment of fish", "treating fish against parasites", "controlling parasites", and similar terms, are intended to refer to prophylactic or responsive treatment, such as the control, elimination, protection against, and/or prevention of the parasitic sea lice infection, infestation, or condition in a fish population. The treatment of fish parasite infestations encompasses reducing the mean number of parasitic sea lice infecting each fish in a fish population. The control of parasitic infestations encompasses preventing an increase in the mean number of parasites infecting each fish in a fish population. These terms also encompass, depending on the condition of the fish, preventing the onset of an infestation with parasitic sea lice, or of symptoms associated with a parasitic infestation, including reducing the severity of a disorder or condition or symptoms associated with the infestation. Thus, "treatment" can refer to administration of diafenthiuron to a fish that is not at the time of administration afflicted with parasitic sea lice. Treating also encompasses preventing the recurrence of a fish parasite infestation or of symptoms associated therewith as well as references to "control" (such as, for example, kill, repel, expel, incapacitate, deter, eliminate, alleviate, minimize, and eradicate).

The fish parasites are sea lice and the above definitions are to be interpreted accordingly.

Reference to treating a parasitic infestation "in" a fish is understood to include treatment of an external parasite, i.e., the sea lice, which feeds "on" a fish and does not necessarily exist inside the fish.

The term "veterinarily acceptable", unless otherwise indicated, indicates that the substance or composition must be compatible chemically and/or toxicologically, with the fish being treated, and/or if applicable, with the other ingredients comprising a composition or formulation for use in treating the fish. The compositions disclosed can be administered in a variety of ways, and the term "veterinarily acceptable" further indicates that the method of administration is not harmful to the fish being treated. It should be noted that the disclosed diafenthiuron compositions and formulations can be administered alone or in combination with one or more veterinarily acceptable carriers, stabilizers, preservatives, colorants, flavourants, excipients, or other agents, as appropriate, and as will be understood by the skilled person.

### Diafenthiuron

References to diafenthiuron refer to diafenthiuron in pure form, or appropriately formulated in a veterinarily acceptable formulation, optionally together with other substances. In addition, references to diafenthiuron refer to diafenthiuron in free form or in the form of a veterinarily acceptable salt.

Despite a strong prejudice in the art that diafenthiuron is highly toxic to fish the present inventors have surprisingly found that diafenthiuron can be administered to fish in parasitically effective amounts without any toxic effects on the fish.

It has also advantageously now been found that diafenthiuron may be used to effectively kill parasitic sea lice. Moreover, diafenthiuron is advantageously effective against sea lice at multiple stages of the louse life cycle.

Diafenthiuron has been found to be effective in treating parasitic sea lice, when used alone, without any requirement for the use of additional parasiticides or other agents.

Diafenthiuron is a thiourea-based insecticide/acaricide. It is also known by the chemical names: N-tert-butyl-N'-[2,6-di(propan-2-yl)-4-phenoxyphenyl]carbonothioic diamide; 1-tert-butyl-3-(2,6-diisopropyl-4-phenoxyphenyl)thiourea; and *N*-[2,6-bis(1-methylethyl)-4-phenoxyphenyl]-*N*'-(1,1-dimethylethyl)thiourea. A number of variations in the spelling of diafenthiuron are frequently used, and in particular, diafenthiuron is often referred to as diafentiuron. For the avoidance of doubt, these different spellings of the word "diafenthiuron" refer to the same compound, the structure of which is shown below:

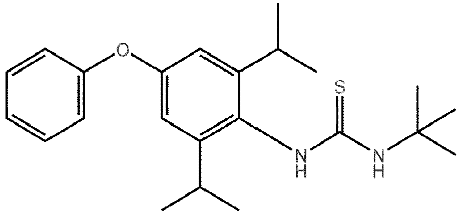

Diafenthiuron is a pro-pesticide and the active metabolite is believed to be the corresponding carbodiimide. Diafenthiuron has been classified in Group 12A under the IRAC Mode of Action Classification Scheme.

Diafenthiuron has previously frequently been described as being toxic to fish, and there is a strong prejudice in the art that diafenthiuron should not be administered to fish, or indeed, allowed to enter waterways. Lethal concentration values (LC₅₀ (96h)) for Rainbow trout of as low as 0.0007mg/l are frequently reported in material safety data sheets for diafenthiuron. Possibly due to this strong prejudice in the art, diafenthiuron has not previously been investigated for use in treating parasitic sea lice in fish.

Despite this widely held prejudice in the art, however, diafenthiuron has now surprisingly been found to be safe both from a toxicological and an environmental perspective. Specifically, it has surprisingly been found that diafenthiuron may be used to effectively treat parasitic sea lice without any toxic effects on the fish.

Diafenthiuron may in general be applied at any stage of the fish development in order to treat parasitic sea lice. For example, in the case of salmon, treatment may take place advantageously whilst the fish are at sea.

Diafenthiuron has previously been described for use as a pesticide in treating crops. However, it has frequently been found that effectiveness of a particular parasiticide compound against one form of parasite (such as a crop pest) is not an indicator of the compound being effective against another type of parasite (such as a fish parasite). For example, only a very limited number of the numerous parasiticide compounds available have shown good efficacy against sea lice. These include the pyrethroids and benzoylureas. There are several factors that contribute to the difficulties experienced when known antiparasitic compounds have been tested on new species of parasite. These include the large genetic and metabolic diversity between the various species of parasites, and the fact that the parasites occupy very different habitats and have evolved different strategies for transmission and infection of the host. In view of this, it is surprising that diafenthiuron has been found to demonstrate advantageously high efficacy in treating parasitic sea lice in fish.

Indeed, diafenthiuron has only previously been reported for use as an insecticide and acaricide. In contrast, many fish parasites, including sea lice, are crustaceans and diafenthiuron could not, therefore, be assumed to be effective in controlling these fish parasites.

Moreover, diafenthiuron has advantageously been found to be highly effective for treating sea lice at different stages in the life cycle of the parasite, including the copepodite, preadult and adult stages. A drawback of many parasiticides that are currently used to treat sea lice (such as, for example, organophosphates) is that they are not effective against the sea lice at all stages of the parasite life cycle. This means that lice at certain stages in the life cycle are not effectively treated by the parasiticide, and hence parasiticide treatment must be repeated several times. Repeating the sea lice treatment is undesirable for many reasons. For example, there is the additional expense associated with the increased requirement for parasiticide and for repeating the treatment procedure, as well as the increased risk of harmful effects to the fish, the farmer, the consumer, and the environment, as a result of increased exposure to these chemicals. There is also an increased risk of the sea lice developing resistance to the parasiticide. A further economic consideration is that the fish cannot be fed directly prior to or during exposure to parasiticide to ensure that the surrounding water is free from food residue and faeces which may interfere with the treatment process. As a result, a single treatment may result in a 1-2% weight "loss"; weight which the fish would otherwise have gained had it not been denied food during the treatment period. This figure is significant on the scale of commercial aquaculture, and represents a significant cost to farmers through lost feeding time. The need to repeat the parasiticide treatment clearly exacerbates this cost. It is therefore a highly advantageous finding that diafenthiuron can be used to treat sea lice at multiple phases of their life cycle, thus reducing or removing the requirement for multiple treatment cycles.

### Fish

Diafenthiuron, or a veterinarily acceptable diafenthiuron salt, may be used to treat parasitic sea lice, in various different types of fish including food fish, breeding fish, aquarium, pond, river, reservoir fish of all ages occurring in freshwater, sea water, and brackish water. For example, bass, bream, carp, catfish, char, chub, cichlid, cobia, cod, eel, flounder, gourami, grayling, groupers, halibut, mullet, pangasius, plaice, pompano, roach, rudd, salmon, sole, tilapia, trout, tuna, whitefish, yellowtails, turbot, blue fin tuna, tench, amberjack, arowana, snakehead, puffers, croaker, rockfish, barramundi, meagre, sturgeon, lumpsucker, wrasse.

Of particular note, the fish that may be treated are of the order Samoniformes, Siluriformes, Perciformes, Cypriniformes, Tetraodontiformes, Osteoglossiformes, Acipenseriformes and Scorpaeniformes .

Diafenthiuron compositions may in particular be suitable for treating salmon. The term "salmon" will be understood as comprising all representatives of the family Salmonidae, especially of the subfamily salmoninae, and preferably, the *Salmo* or *Oncorhynchus* genus. In particular, diafenthiuron may be used to treat parasiticsea lice in any of the following fish: the Atlantic salmon (Salmon salar), rainbow trout (Oncorhynchus mykiss), brown or sea trout (S. trutta), the Pacific salmon: Cherry salmon or seema (O. masou), Taiwanese salmon (O. masou formosanum), chinook salmon or King salmon (O. tshawytscha), chum salmon or Calico salmon (O. keta), coho salmon or silver salmon (O. kisutch), pink salmon (O. gorbuscha), Sockeye salmon or Red salmon (O. nerka), artifically propagated species, such as Salmo clarkii, and Salvelinus species such as Brook trout (S. fontinalis) .

Fish that may be treated in particular include Atlantic and Pacific salmon and the sea trout.

Diafenthiuron compositions may in particular also be suitable for treating other types of fish, including tilapia.

The aquatic environment in which the fish may be treated may be freshwater or preferably a saltwater (e.g. marine) environment. Saltwater environments include, for example, the sea, inlets, saltwater lakes, rivers or lochs.

### Parasites

In the present context, the term "fish parasite" is intended to refer to any organism that lives on or in fish, or otherwise benefits at the expense of the host fish, for example, any organism that obtains some or all of its nutritional requirements from the fish. Parasites include both ectoparasites, that live or feed on the outer surface of the fish, and endoparasites, that live inside the body of the fish.

Diafenthiuron formulations may be used in the treatment of fish against parasites. This includes, in particular, parasites of the order Siphonostomatoida (lice), Dactylogyridea (Diplectanum), Mazocraeidea (such as Sparycotyle,and Heterobothrium), Hymenostomatida (freshwater white spot), Capsalidae (Benedenia), Dactylopodida (P. perurans), Cyclopoida, Parabodonida (Cryptobia spp.), Scuticocilitida (ciliates), Gluegeida (L. salmonae), Bivalvulida (such as Myxobolus, Ceratomyxa and H. ictaluri), Monopisthocotylea (gyrodactylus), Strigeatida (blood flukes), Botriocephalidea (tapeworms), Spirurida (nematodes), Arguloida (carp lice in freshwater) and Ascaridida (Anisakis) and genus Cryptocaryon (white spot in seawater), Diplostomum (eye fluke in freshwater), and Enteromyxum (E. leei). The fish parasite may, in particular, be any parasitic marine crustacean.

Parasiticides which are currently used to combat fish parasites include, for example, organophosphates, pyrethroids, emamectin benzoate, hydrogen peroxide or benzoylureas. Not all of these parasiticides have always been available, complicating resistance control programs. Shortcomings associated with currently used parasiticides include the high concentrations in which they have to be used, the ecological problems associated with the use of the chemicals, the capacity of fish parasites such as sea lice to develop resistance, and the increasing prevalence of resistance. Surprisingly, diafenthiuron has been found to be effective for treating fish parasites.

Fish parasite infestations may be measured as the percentage of infected fish (i.e. the percentage of fish having at least one parasite). By means of the claimed use of diafenthiuron, fish parasite infestation may be reduced by at least about 10%, 20%, 30%, 40%, 50%, 60%, 70%, 80%, 90%, 95% or 99%. Preferably, the reduction may be at least about 30% or 50%.

Fish parasite infestations may also be measured as the number of fish parasites per fish (i.e. the mean load). By means of the claimed use of diafenthiuron, the number of parasites per fish may be reduced by at least about 10%, 20%, 30%, 40%, 50%, 60%, 70%, 75%, 80%, 90%, 95% or 99%. Preferably, the reduction may be at least about 40% or 60%.

### Sea Lice

Diafenthiuron, or a veterinarily acceptable diafenthiuron salt, may be used for treating sea lice in fish. Sea lice are ectoparasites found in freshwater, sea water, and brackish water. The sea lice may be of the Caligidae family, including in particular, parasites of the genera *Dissonus, Caligus* (including *C*. *curtus, C. elongatus, C. clemensi, C. rogercresseyii),* and *Lepeophtheirus* (including *L. salmonis).*

Sea lice are major parasites of farmed fish, particularly on salmonids, and, when present in high numbers, can cause serious disease and ultimately host death. It is also important to keep the infestation rates low to prevent infestation of migrating wild salmonids. In fish farms, where highly concentrated fish populations are present, sea lice can have a devastating effect on the stock. *Lepeophtheirus salmonis* is one of the most common species of sea lice, and sea lice of the *Caligus* species are also problematic for the fish industry. It is believed that sea lice affect salmon by feeding on the mucus and skin, and sucking blood. In addition, it appears that the lice have immunomodulatory effects on the host fish and are able to function as a vector in the transmission of other fish diseases.

Mortality rates of over 50%, based on sea lice infestation, have been reported from Norwegian fish farms. In a first phase, sea lice infestation is seen in the appearance of the parasites attached to the fish, and later from the damage caused to skin and tissue. The most severe damage is observed in smolts which are just in the phase in which they have changed from fresh water to sea water and in broodstock fish which have stopped feeding.

Sea lice of the species *Lepeophtheirus* are particularly harmful to fish because even a small number of the parasites can cause widespread tissue damage. The life cycle of *Lepeophtheirus* consists substantially of three free-swimming larval stages (nauplius I & II and copepodid stages), the copepodids attach to the fish and develop through two chalimus stages, two pre-adult stages and the actual adult stage. The chalimus and adult stages are host-dependent. The most dangerous stages, because they cause the greatest damage, are all those parasitizing on the fish, especially the adult stages.

Surprisingly, diafenthiuron has been found to be effective for treating sea lice, and is effective against multiple different stages in the sea lice life cycle. In particular, diafenthiuron has been found to be highly effective at treating sea lice during the infective stages of *L. salmonis.*

### Toxicity

Despite a significant prejudice in the art, it has now surprisingly been found that diafenthiuron may be used to treat parasitic sea lice in fish, and is effective at a concentration that is not toxic to the fish.

Formulations comprising diafenthiuron for use in treating parasitic sea lice in fish are not toxic when administered to fish. Specifically, the diafenthiuron formulations do not induce excessive mortality in the treated fish population.

Generally, the toxicity of a formulation that is administered to fish is related to a number of factors, which may include, for example, the size and weight of the fish, whether the fish are in freshwater or saltwater, and the developmental stage of the fish. As a result, the concentration of diafenthiuron that may be used without inducing excessive mortality may differ, depending on the nature and habitat of the fish population to be treated. For example, larger fish may be able to tolerate greater amounts and concentrations of diafenthiuron than smaller fish. Formulations comprising diafenthiuron for use in the invention are non-toxic and preferably produce no mortality.

Toxicity in a commercial setting may be measured by any applicable method, and suitable methods are known to the skilled person. For example, the formulation, which is not toxic to fish, preferably produces no mortality in the large size commercial fish population that has been treated. At most, the formulation may induce 2% mortality in the large size commercial fish population that has been treated. More preferably, the level of mortality is less than 1%, such as less than 0.5% compared to a control treated group.

As the skilled person will be aware, the toxicity of a formulation can be determined in a number of ways. Some methods may involve trials involving large numbers of fish. Therefore, to avoid the need to test large numbers of fish, a model test for toxicity of the formulations involving much smaller numbers may be used. The use of a model test, such as one of those described below, provides the further advantage that it removes complicating factors such as the size and sensitivity of the fish tested, because small fish are often more susceptible to a given treatment dosage than larger fish. In this way, the model test allows a standardised comparison of the toxicity of different concentrations and formulations of diafenthiuron to be obtained.

In a model test for the toxicity of a diafenthiuron formulation that is administered by injection, a small number of Atlantic salmon, such as 30 fish per group, are treated at a water temperature of about 17 degrees C. One group of fish is treated with a large dosage volume (0.2ml) of the subject diafenthiuron formulation, and a second group is treated in the same way with a regular dosage volume (0.05ml) of the subject diafenthiuron formulation. Additionally, two control groups are treated in an identical manner to the subject groups, but with a control formulation, such as PBS, instead of the subject diafenthiuron formulation. The fish are monitored and the accumulated mortality over seven days is recorded. The subject diafenthiuron formulation is then considered to be non-toxic if two conditions are met. The first condition is that there is no increased mortality in the subject group versus the relevant control group when the diafenthiuron formulation is administered in an amount of 0.05ml. The second condition is that there is at most a 10% increase in mortality in the subject group versus the relevant control group when the diafenthiuron formulation is administered in an amount of 0.2ml.

In a model test for the toxicity of a diafenthiuron formulation that is administered by bath treatment, a small number of Atlantic salmon, such as 5 fish, are treated for 60 minutes at a water temperature of about 8-12 degrees C. The subject diafenthiuron formulation is then considered to be non-toxic if none of the fish die as a result of the treatment.

### Use of Diafenthiuron

To be an effective parasiticide, the tolerance of the host to the parasiticide must be greater than the tolerance of the parasite to the parasiticide. The difference in tolerance may be due, for example, to differences between the uptake, genetics, and/or metabolism of the host and parasite species. The variance in tolerance can be used to find a suitable therapeutic window, i.e. a concentration that inactivates the parasite without harming the host excessively. It has now advantageously been found that a therapeutic window exists in which diafenthiuron may be used to effectively and efficiently treat parasitic sea lice in fish, but is not toxic to the fish.

The term "therapeutically effective amount" refers to an amount of diafenthiuron that (i) treats the fish parasite infection or infestation, (ii) attenuates, ameliorates, or eliminates one or more symptoms of the infection or infestation, and/or (iii) prevents or delays the onset of one or more symptoms of the fish parasite infection or infestation.

Fish may be treated with parasiticides periodically based on infestation rates. In this case, treatments may be administered more frequently in the summer because parasitic sea lice generally develop more quickly at higher water temperatures. In order to optimise treatment, farmers may coordinate the timing and type of treatment used. The finding that diafenthiuron is suitable for use in treating parasitic sea lice provides a further option for arranging treatment strategies.

Within the treated fish, diafenthiuron is preferably distributed in a pharmacologically effective concentration to all tissues and organs, including mucus, skin, gills, and intestines. The fish preferably absorbs the diafenthiuron, and the concentration of diafenthiuron is preferably maintained and detectable at a therapeutically effective concentration within the blood, fillet and skin of the fish for an extended period of time. Thus, in addition to a direct treatment effect, the diafenthiuron formulation preferably also protects fish from new attacks by parasites for an extended period of time after administration.

When using diafenthiuron for treating fish parasites, the fish will absorb the diafenthiuron such that a therapeutically effective concentration of diafenthiuron will be maintained for a prolonged period, for example for at least 5 months, preferably for at least 6 months and more preferably for at least 9 months.

Diafenthiuron has low solubility and is miscible with water. Generally, the diafenthiuron formulations for use in treating parasitic sea lice may be in the form of liquid compositions, in pure form, and/or as a solid active substance (for example, in a specific particle size). Preferably, the diafenthiuron formulation may comprise at least one of the adjuvants which are conventionally used in formulation technology, such as solvents, excipients, solubilisers, etc. The formulations may prepared in a manner known per se, typically by mixing, granulating and/or compacting the diafenthiuron with solid or liquid carriers, where appropriate with the addition of further adjuvants, such as emulsifiable or dispersing agents, stabilisers, colourants, antioxidants and/or preservatives.

### Bath Treatment

Diafenthiuron may be administered to fish by bath treatment, for example by placing the fish into a "medicinal bath" and keeping them there for a period of time (minutes to several hours), for example, when being transferred from one net pen or breeding basin to another. It is also possible to treat the biotope of the fish temporarily or continuously, such as, for example, the net cages, entire ponds, aquaria, tanks or basins in which the fish are kept. For use as a bath treatment, diafenthiuron or a formulation comprising diafenthiuron may be dissolved or suspended in the water containing the fish and parasite.

Diafenthiuron may be used at a concentration of from about 0.01 ppm to about 100 ppm, preferably about 0.05 ppm to about 50 ppm, more preferably about 0.1 ppm to about 20 ppm, and even more preferably from about 0.25 ppm to about 10 ppm, or about 0.5 ppm to about 5 ppm, such as about 1 ppm, 2 ppm, or 3 ppm, based on total bath volume. These values represent the concentration of treatment agent in the water containing the fish (i.e. the actual levels needed to control the parasite population). The concentrations are achieved by adding a concentrated stock of diafenthiuron, such as a stock solution or formulation to the enclosure containing the fish. The skilled person is able to determine how much diafenthiuron should be added from knowledge of the volume of the enclosure containing the fish and the concentration of the stock solution.

The concentration of diafenthiuron during application depends on the manner and duration of treatment and also on the age and condition of the fish being treated. A typical immersion time ranges from about 15 minutes to about 4 hours, in particular from about 30 minutes to about 1 hour. Diafenthiuron can be dissolved or suspended in the surrounding water containing the fish and parasite. The bath can contain further common excipients known in the art for preparing aqueous solutions, for example, stabilizers, antifoaming agents, viscosity modifiers, binders, and tackifiers.

This method of administration is very simple and labour efficient, and has the added advantage that the diafenthiuron may be able to act directly on sea lice parasites that are predominantly external to the fish.

Diafenthiuron for bath application or for treating the biotope may be prepared or formulated in any suitable form, such as powder, granulate, solution, emulsion, micro/nanoemulsion, emulsifiable concentrate, suspension, nanosuspension, or suspension concentrate, tablet or the simply pure diafenthiuron itself. The user may use these formulations in diluted or undiluted form. The most suitable form will be known to the skilled person and may dependent on the exact circumstances of the administration, such as the degree of dilution, the stability of the composition, etc.

Diafenthiuron formulations for administration to fish by bath treatment may be in liquid form, and may be prepared in any suitable manner. For example, formulations may be prepared by dissolving diafenthiuron under stirring at ambient temperature in a premix comprising solvent and/or any additional agents, such as a solubilizer and/or an excipient, as required. In example formulations for administration by bath treatment, diafenthiuron formulations may be prepared by dissolving diafenthiuron in a premix comprising acetone and/or Pharmasolve as a solvent and Kolliphor EL as a solublizer.

It is also possible to use semi-solid formulations for the bath treatment. The active substance, which is suspended or dissolved in oily or fatty matrices, is washed out. The release can be controlled by the choice of adjuvants, concentration of the active substance and form. Melts of hard fats comprising the active substance are also suitable for use.

The diafenthiuron formulation may be used in a water-soluble packaging, such as in a bag comprising polyvinyl alcohol. In this case the user is not exposed to the active substance or its formulation. In practice it is also possible to use, for example, those forms of application where the active substance is contained in a readily water-soluble matrix of a film, or in films from which it diffuses over the period of application.

### Feed

The fish may be treated orally, for example via an in-feed treatment, wherein the composition is added to the feed provided to the fish.

Reference to "fish food", "fish feed", and "feed composition" indicates substances specially adapted for oral administration to fish. Particularly, a food substance comprising at least one of fats, nutrients, protein, vitamins, minerals, and carbohydrates in liquid, flake, granule, or pellet form, which is capable of adsorbing or being admixed with diafenthiuron or a diafenthiuron composition. Preferably, the fish food includes diafenthiuron and at least one of corn starch, pregelatinized corn starch, protein, nutrients, vegetable oil and/or fish oil.

Formulations of diafenthiuron for oral administration may take the form, for example, of powders, premixes, granulates, solutions, emulsions, micro/nanoemulsions, emulsifiable concentrates, suspensions, nanosuspensions or suspension concentrates which are mixed homogeneously as feed additives with the feed, or powders, premixes, granulates, solutions, emulsions, micro/nanoemulsions, emulsifiable concentrates, suspensions, nanosuspensions or suspension concentrates which are administered in the form of pills, the outer coat of which can consist e.g. of fish feed compositions which cover the active substance completely.

A diafenthiuron-medicated fish feed may be prepared by incorporating a suitable amount of diafenthiuron or a salt thereof into the fish feed product. The diafenthiuron may be incorporated into the feed mixture prior to pelleting. Alternatively, pellets or granules of fish feed may be coated with diafenthiuron. For example, commercially available fish pellets or granules may be coated with a pre-mix containing the diafenthiuron and one or more suitable excipients such as a starch, fumed silica (Aerosil®), microcrystalline cellulose, lactose or the like. In addition, a typical preservative may be present.

The feed pellets may be coated with the pre-mix by a dry-coating method. To this end the pre-mix is mixed with the pellets so that it is uniformly distributed onto the pellets; preferably, fish oil or vegetable oil is then added to the mixture to coat the medicated pellets. In an alternative, the pre-mix is first mixed with fish or vegetable oil, which is then mixed with the pellets to disperse it onto them, and additional fish or vegetable oil is added to the coated pellets and mixed until the pellets are thoroughly coated. In a further alternative, the pre-mix is first dispersed in some fish or vegetable oil, said dispersion is then sprayed onto the pellets to disperse it onto them under a vacuum coating system and mixed until the pellets are thoroughly coated.

Following the addition of the active ingredient to the fish feed, the pellets or granules comprise, for example, from 0.1-10 g/kg feed, preferably 0.5-5 g/kg feed, or 0.1-10 % (w/w), preferably from 0.25-7.5 % (w/w), and in particular from 0.5-5 % (w/w) diafenthiuron, based on the entire weight of the fish feed.

Diafenthiuron may be administered according to any suitable treatment regime. For example, diafenthiuron may be administered to the subject fish daily, for a period of time preferably of 3 to 13 days, more preferably of 5 to 9 days, and in particular of 7 days.

The fish may be treated with a dose corresponding to 2-15 mg/kg fish, 3-12 mg/kg more specifically 4-10 mg/kg fish.

### Intraperitoneal injection

Treatment with diafenthiuron may also be carried out parenterally; for example, the treatment may comprise the injection of a liquid or suspension formulation of diafenthiuron into the fish to be treated.

Intraperitoneal injection involves injecting a formulation onto the surface of the internal organs of the fish. As a result, the effects, and in particular, the toxicity of injected formulations are difficult to predict, and may be different when the composition is delivered to the fish by intraperitoneal injection in comparison to administration by other means, or to different animals.

However, the water-solubility of diafenthiuron is very low, and has been found to be about 0.06 mg/ml. As a result, if diafenthiuron is prepared in the form of an aqueous solution for intraperitoneal injection it delivers poor performance because the concentration of diafenthiuron is very low due to the low solubility of diafenthiuron in water.

In order to address this problem, diafenthiuron may be used for intraperitoneal injection, or other route of administration, in the form of an aqueous formulation comprising a suspension of nanoparticles. However, there are drawbacks with formulations comprising a suspension or emulsion. For example, in a water-in-oil emulsion in which particulate diafenthiuron is dispersed in the continuous oil phase, the diafenthiuron particles may sediment during production, storage and use, leading to an inhomogeneous product. Moreover, the opacity of the emulsion may hide the extent of the sedimentation.

Alternatively, diafenthiuron may be formulated using a suitable solvent. Because diafenthiuron is insoluble in water a polar solvent such as DMSO may be used but it has been found that polar solvents can be toxic to fish if administered by means of intraperitoneal injection. Therefore, in order to substantially reduce or eliminate the toxic effects of the polar solvent, a further agent such as a solubilizer or a hydrophilic polymer must also be included in the formulation, if the formulation is to be administered by intraperitoneal injection.

As an alternative to a polar solvent, hydrophilic polymers have now surprisingly been found to be both non-toxic for intraperitoneal injection to fish and suitable for dissolving parasiticides. Thus, diafenthiuron formulations may be produced using a hydrophilic polymer without the need for any additional agents, and in particular, without the need for a polar solvent or a solubilizer, which may be advantageous if the formulation is to be administered by intraperitoneal injection as it avoids the possibility of toxic effects of the polar solvent.

Diafenthiuron formulations for intraperitoneal injection to fish may be administered according to any suitable regime. For example, in the case of salmonids, one treatment schedule comprises treating the fish during the initial fresh water phase before transfer to sea. In addition, or alternatively, the treatment may be performed whilst the fish are already at sea.

Diafenthiuron formulations for intraperitoneal injection to fish may be prepared in any suitable manner. For example, formulations may be prepared by dissolving diafenthiuron under stirring at ambient temperature in a premix comprising solvent and/or any additional agents, such as excipients, as required.

Generally, the total volume of a diafenthiuron formulation that may be injected into the fish is about 0.01-2ml, such as, for example, 0.05ml, or 0.1ml. The overall amount of diafenthiuron that may be injected into the fish is preferably 0.2 to 120 mg/kg fish biomass, 0.5 to 100 mg/kg, or 1 to 75 mg/kg of fish biomass, such as 5-50, or 10-30 mg/kg of fish biomass. As a result, diafenthiuron formulations for use in treating fish parasites by intraperitoneal injection generally comprise diafenthiuron in an amount of about, or at least, 10, 15, 20, 25, 30, 35, 40, 45, 50, 55, 60, 65, 70, 75, 80, 85, 90, 95, or 100 mg/ml.

In one example, a diafenthiuron formulation for use in treating parasitic sea lice in fish by intraperitoneal injection comprises: diafenthiuron; a non-aqueous polar solvent, which may be DMSO; a solubilizer, which may be Cremophor EL; and an excipient, which may be propylene glycol. In this example formulation, diafenthiuron may be present in an amount of 10-300 mg/ml, such as 20-250, 30-200, 40-150, 50-120, 70-115, 80-110, 85-105, most preferably 90-100 mg/ml, such as about 90 mg/ml or about 100 mg/ml.

In another example, a diafenthiuron formulation for intraperitoneal injection comprises: diafenthiuron; and a hydrophilic polymer, which may be a PEG or a PEG derivative, such as PEG300 or PEG400 in an amount of up to 50%, 60%, 70%, 80%, 90%, or 100% by weight. The formulation preferably also includes a stabiliser, which may be citric acid, in an amount of 0.1-0.5%, or 0.2-0.4%, most preferably about 0.3%. In this example formulation, diafenthiuron may be present in an amount of 10-150 mg/ml, such as 15-120, 20-100, 25-90 or 30-80 mg/ml.

In another example, a diafenthiuron formulation for intraperitoneal injection comprises: diafenthiuron; a non-aqueous polar solvent, which may be DMSO; a solubilizer, which may be Cremophor EL; and a hydrophilic polymer, which may be a PEG or a PEG derivative, such as PEG300 or PEG400. In this example formulation, diafenthiuron may be present in an amount of 10-300 mg/ml, such as 20-250, 30-200, 40-150, 50-120, 70-115, 80-110, 85-105, most preferably 90-100 mg/ml, such as about 90 mg/ml or about 100 mg/ml.

In yet another example, a diafenthiuron formulation for intraperitoneal injection comprises: diafenthiuron; a non-aqueous polar solvent, which may be DMSO; and a hydrophilic polymer, which may be a PEG or a PEG derivative, such as PEG300 or PEG400. In this example formulation, diafenthiuron may be present in an amount of 10-300 mg/ml, such as 20-250, 30-200, 40-150, 50-120, 70-115, 80-110, 85-105, most preferably 90-100 mg/ml, such as about 90 mg/ml or about 100 mg/ml.

### Polar Solvent

For the avoidance of doubt, as used herein the term "polar solvent" does not encompass the use of water as a solvent, and for clarity, the polar solvent may therefore be referred to as a "non-aqueous polar solvent". A solvent is considered to be a polar solvent if the solvent molecules have a permanent separation of positive and negative charges, or the centres of positive and negative charges do not coincide. Polar solvents have high dielectric constants, for example, a solvent with a dielectric constant above about 15.0 is generally considered to be a polar solvent.

Because diafenthiuron has a low solubility in water, diafenthiuron formulations, particularly formulations for intraperitoneal injection, are preferably non-aqueous and no water is added during the production of the formulations. As the skilled person would appreciate, however, it is nevertheless possible that a small amount of water may unavoidably be present in the formulation, for example as a result of being adsorbed from the atmosphere. Preferably, diafenthiuron formulations for intraperitoneal injection are non-aqueous formulations and contain at most 4%, 3%, or 2%, preferably at most 1.5%, 1% or 0.5% water. In the present context, the term "non-aqueous" refers to formulations which include at most only a very small amount of water, which is not added to the formulation, but may be derived, for example, from the atmosphere. For example, some non-aqueous polar solvents, such as DMSO, are known to be hygroscopic under certain conditions and may therefore cause small amounts of water to accumulate in the formulation, or in the solvent prior to the production of the formulation. For the purposes of clarity, preferred diafenthiuron formulations comprise no water other than that which may be present in the constituent agents due to adsorption.

Examples of polar solvents that may be used individually or in combination in diafenthiuron formulations, such as diafenthiuron formulations for intraperitoneal injection, include acetone, tetraglycol, ethyl acetate, DMF, DMA, DMSO, NMP, ethanol, isopropanol, glycerin, ethylene glycol, diethylene glycol, 1-propanol, 1-butanol, 2-butanol, acetonitrile and methanol. In particular, the polar solvent may be a polar aprotic solvent such as DMF, DMA, or DMSO.

Generally, when the polar solvent is a polar aprotic solvent such as DMSO, diafenthiuron formulations for intraperitoneal injection comprise about 5-40% (w/w) polar solvent. Preferably, such formulations comprise less than 35%, less than 30%, less than 25%, or less than 20% (w/w) polar solvent, such as, for example, 7-35%, 10-30%, 12-25% or 15-20% (w/w) polar solvent, such as about 5%, 10%, 15%, 20%, or 25% (w/w) polar solvent.

Diafenthiuron formulations for administration to fish by other routes, such as for administration by bath treatment, may comprise more than 40% polar solvent, such as up to 50%, 60%, 70%, 80%, 90%, or 100% polar solvent.

In some embodiments, diafenthiuron may be solubilized in a combination of a polar solvent and solubilizer, such as acetone or DMSO and Kolliphor EL. The resulting liquid may then be mixed with water.

### Solubilizer

Diafenthiuron formulations for use in treating parasitic sea lice, by intraperitoneal injection in particular, may comprise a solubilizer.

In the present context, the term "solubilizer" refers to any substance that is capable of increasing the solubility of diafenthiuron, for example when it is used in a polar solvent, a mixture of polar solvent and excipient, or a mixture of polar solvent and hydrophilic polymer. The solubilizer may be an amphiphilic molecule or a surfactant, and could be cationic, anionic or non-ionic, non-polymeric or polymeric.

Solubilization is a process in which the solubility of a compound is increased by the formation of micellar structures after addition of a solubilizer. The term solubilisation is almost exclusively used for aqueous systems. In aqueous pharmaceutical formulations, for instance, solubilizers are often used to facilitate the dissolution of poorly soluble compounds. A solubilizer may be used in the manufacture of an aqueous diafenthiuron formulation, for example for use in a bath treatment.

In non-aqueous formulations, on the other hand, such as those preferred for administration by intraperitoneal injection, solubilizers are mainly added in order to have a function during the use of the products in aqueous media and not in the formulation *per se.* While the process of solubilization is well described in aqueous systems, solubilization in non-aqueous systems *per* se is less well understood. Basically, there is no clear rational reason for adding a solubilizer to a non-aqueous formulation, unless the solubilizer has a function during use in aqueous media as described above. Hence, in non-aqueous formulations such as those described above in respect of diafenthiuron formulations for intraperitoneal injection, the use of a solubilizer would not be considered. It has now been surprisingly and advantageously discovered that diafenthiuron formulations suitable for intraperitoneal injection may be produced comprising a non-aqueous polar solvent, and that the level of non-aqueous polar solvent required in the formulation may be reduced below toxic levels if a solubilizer is also included.

Various solubilizers may be used in the formulation, and for example, the solubilizer may be selected from the list of solubilizers or emulsifiers given in the Handbook of pharmaceutical additives, M. Ash and I. Ash, Gower publishing limited, 1996, page 900-903 or 874-880, respectively. Suitable solubilizers include, for example: polyoxyethylene sorbitan fatty acid esters such as polysorbate 20 (Tween 20), polysorbate 21, polysorbate 40, polysorbate 60, polysorbate 61, polysorbate 65, polysorbate 80 (Tween 80), polysorbate 81, polysorbate 85 and polysorbate 120; polyoxyethylene castor oil derivatives such as Polyoxyl 5 castor oil, Polyoxyl 9 castor oil, Polyoxyl 15 castor oil, Polyoxyl 35 castor oil (Cremophore EL), Polyoxyl 40 castor oil, Polyoxyl 40 hydrogenated castor oil, polyoxyl 60 castor oil, Polyoxyl 60 hydrogenated castor oil, Polyoxyl 100 castor oil, Polyoxyl 100 hydrogenated castor oil, Polyoxyl 200 castor oil and Polyoxyl 200 hydrogenated castor oil; poloxamers (polyoxyethylene - polyoxypropylene copolymers) such as poloxamer 124, poloxamer 188, poloxamer 237, poloxamer 338 and poloxamer 407; polyoxyethylene alkyl ethers such as Cetomacrogol 1000, Polyoxyl 6 cetostearyl ether, Polyoxyl 20 cetostearyl ether, Polyoxyl 25 cetostearyl ether, Polyoxyl 2 cetyl ether, Polyoxyl 10 cetyl ether, Polyoxyl 20 cetyl ether, Polyoxyl 4 lauryl ether, Polyoxyl 9 lauryl ether, Polyoxyl 23 lauryl ether, Polyoxyl 2 oleyl ether, Polyoxyl 10 oleyl ether, Polyoxyl 20 oleyl ether, Polyoxyl 2 stearyl ether, Polyoxyl 10 stearyl ether, Polyoxyl 21 stearyl ether and Polyoxyl 100 stearyl ether; polyoxylglycerides such as Caprylocaproyl polyoxylglycerides, Lauroyl polyoxylglycerides, Linoleoyl polyoxylglycerides, Oleoyl polyoxylglycerides and Stearoyl polyoxylglycerides; and sorbitan fatty acid esters like Sorbitan diisostearate, Sorbitan dioleate, Sorbitan monoisostearate, Sorbitan monolaurate, Sorbitan monooleate (Span 80), Sorbitan monopalmitate, Sorbitan monostearate, Sorbitan sesquiisostearate, Sorbitan sesquioleate, Sorbitan sesquistearate, Sorbitan triisostearate, Sorbitan trioleate and Sorbitan tristearate; macrogol 15 hydroxystearate; cetostearyl alcohol and cetyl alcohol.

Solubilizers that have been found to be particularly suitable for use in the manufacture of diafenthiuron formulations include, for example, Tween, Brij C10, Kolliphor HS15, Kolliphor EL, or Cremophor RH40. Especially preferred solubilizers include Tween 80 and Cremophor EL.

When the diafenthiuron formulation comprises a solubilizer, the solubilizer is generally used in the amount of about 5-60% (w/w). Preferably, the formulation comprises 10-55% (w/w) solubilizer, more preferably 15-50% (w/w) solubilizer, such as, for example, about 25, 30, 35, 40, 45, 50 or 55% (w/w) solubilizer. The diafenthiuron formulation may comprise a mixture of two or more different solubilizers.

When the formulation comprises Cremophor EL as the solubilizer, it is preferably present in the formulation in an amount of 30-60%, such as about 35, 40, 45, 50 or 55%.

### Hydrophilic Polymer

Diafenthiuron formulations for use in treating parasitic sea lice, by intraperitoneal injection in particular, may comprise a hydrophilic polymer.

Hydrophilic polymers contain polar or charged functional groups, rendering them soluble in water and/or in polar solvents. Various hydrophilic polymers may be used in diafenthiuron formulations, optionally in combination with a non-aqueous polar solvent and/or solubilizer, or in the absence of any other solvent or solubilizer. Diafenthiuron formulations for intraperitoneal injection of fish may comprise a hydrophilic polymer and optionally also a polar solvent.

The hydrophilic polymer may be a polymer that is capable of hydrogen bonding.

Preferably the hydrophilic polymer has an average molecular weight of 50-2000, such as between 100-1800, or 150-1600.

The hydrophilic polymer may be a polyalkoxylated alcohol, or a derivative of a polyalkoxylated alcohol. Preferably, the hydrophilic polymer is a polyalkoxylated diol or a derivative of a polyalkoxylated diol. For example, the hydrophilic polymer may be a polyalkylene glycol or a derivative of a polyalkylene glycol.

Suitable hydrophilic polymers include, in particular, PEGs and PEG derivatives and PPGs and PPG derivatives.

Examples of PEG derivatives that may be used include: monofunctional PEGs, such as poly(ethylene glycol) methylether; homobifunctional PEGs, such as poly(ethylene glycol) dimethylether, poly(ethylene glycol) diglycidyl ether, or poly(ethylene glycol) bis(carboxymethyl)ether; heterobifunctional PEGs, such as poly(ethylene glycol) tetrahydrofurfuryl ether; multi-arm PEGs or star-PEGs, such as trimethylolpropane ethoxylate, or glycerol ethoxylate.

Examples of PPG derivatives that may be used include: monofunctional PPGs, such as poly(propylene glycol) monobutyl ether; and homobifunctional PPGs, such as poly(propylene glycol) diglycidyl ether.

The hydrophilic polymer may be a polyether such as glycerol propoxylate.

Preferably the hydrophilic polymer is PEG or a PEG derivative. For example, the hydrophilic polymer may be PEG having a molecular weight of between 200 and 1000. Preferably the hydrophilic polymer is PEG300, PEG400, or PEG600.

The hydrophilic polymer may comprise a combination of two or more hydrophilic polymers.

Preferably the hydrophilic polymer is not toxic to fish, particularly when administered by intraperitoneal injection.

Diafenthiuron formulations may comprise a hydrophilic polymer as the only solvent. In this case, the hydrophilic polymer may be present in the formulation in an amount of 5-100% by weight.

The hydrophilic polymer may be present in the diafenthiuron formulation in combination with another solvent, such as a non-aqueous polar solvent. In this case, the hydrophilic polymer may be present in the formulation an amount of 5-95%, 10-90, 15-85, 20-80, 25-75, 30-70, 35-65, 40-60, 45-65, or 50-60% by weight.

### Excipient

Diafenthiuron formulations for use in treating parasitic sea lice may comprise an excipient. In the present context, the term "excipient" is used to refer to any inert, non-toxic substance which is added to the formulation, for example, as a diluent to allow the diafenthiuron formulation to be prepared to the desired consistency, form or concentration.

Any suitable excipient may be used in diafenthiuron formulations, and the formulations may comprise one or a combination of excipients. For example, the excipient or mixture of excipients could be selected from, but not restricted to, the list of carriers/vehicles or solvents given in Handbook of pharmaceutical additives, M. Ash and I. Ash, Gower publishing limited, 1996, page 862-863 or 903-904, respectively. Excipients that are suitable for use in diafenthiuron formulations include, for example, polar, low-molecular weight substances like propylene glycol, butylene glycol, propylene carbonate, ethanol, isopropyl alcohol, glycerol, butanol and ethyl lactate.

Excipients for use in diafenthiuron formulations may include one or more polar oils. Polar oils contain heteroatoms that give the molecules a dipole moment, and this provides the substances with unique solubility properties when compared with nonpolar oils such as mineral oil. Polar oils which may be suitable for use in diafenthiuron formulations include, for example: fatty alcohols such as myristyl alcohol, oleyl alcohol and stearyl alcohol; esters such as isopropyl myristate, isopropyl palmitate and ethyl oleate; triglycerides such as tricaprylin, medium-chain tryglycerides and triolein; and coconut oil.

Generally, diafenthiuron formulations may comprise about 5-70% (w/w) of excipient. Preferably one or a combination of excipients is included in the formulation in a total amount of 10-60% (w/w), 20-55% (w/w), or 30-50% (w/w), such as about 35, 37.5, 40, 42.5, or 45% (w/w).

### Stabilisers

Diafenthiuron formulations for use in treating parasitic sea lice may include a stabiliser. The stabiliser may be a pH control agent, such as, for example, citric acid. As the skilled person would appreciate, the term "pH" only makes sense for aqueous media. Some of the disclosed diafenthiuron formulations, however, particularly diafenthiuron formulations for intraperitoneal injection of fish, may be non-aqueous formulations. Therefore, in the present context the terms "pH control agent", "pH modifier", and similar terms, are used to refer to any substance, which, when used in an aqueous solution, is potentially capable of controlling the pH, such as buffering and/or adjusting the pH. Such agents have surprisingly been found to increase the chemical stability of diafenthiuron in non-aqueous formulations.

A pH control agent may be included in diafenthiuron formulations in an amount of up to about 3 volume%, such as 0.05-2.0 volume%, or 0.1-1.0 volume%. Preferably, the pH control agent may be included in the formulation in an amount of about 0.1, 0.2, 0.3, 0.4, or 0.5 volume%, and most preferably may be included in an amount of 0.3 volume%.

Various stabilisers, which may be pH control agents, may be used in the formulation. For example, the pH control agent for use in the formulation may be selected from, but not restricted to, the list of pH control agents given in Handbook of pharmaceutical additives, M. Ash and I. Ash, Gower publishing limited, 1996, page 896. Specifically, suitable pH control agents for use in the formulation have been found to include, for example, organic acids like citric acid, acetic acid, adipic acid, hydrochloric acid, N-hydroxysuccinic acid, lactic acid, malic acid, oxalic acid, propionic acid, ricinoleic acid, succinic acid and tartaric acid.

### Other agents

Formulations of diafenthiuron for use in treating parasitic sea lice may include additional agents.

For example, when the diafenthiuron formulation is a liquid for intraperitoneal injection in particular, the formulation may comprise a freezing point lowering agent. Suitable freezing point lowering agents include, for example, propylene glycol and ethanol.

In addition, or alternatively, the liquid diafenthiuron formulation may comprise a preservative. Any suitable preservative or combination of preservatives may be used, including, for example, chlorobutanol, benzyl alcohol, a paraben (such as methylparaben or propylparaben), sorbic acid, phenoxyethanol, or thiomersal. The preservative may be included in the formulation in an amount of from 0.001 % to 2.5 % (w/v), such as from 0.002 to 1.5% (w/v).

### Combination Formulations

Diafenthiuron may be used alone for use in treating parasitic sea lice. Alternatively, diafenthiuron may be used in a combination formulation together with one or more additional active agents. References to "additional active agent(s)", unless otherwise indicated, refer to other antiparasitic compounds or products, antigens, adjuvants, inactivated or killed viruses or bacteria, and the like, that provide a therapeutically effective amount of said agent(s) that are useful for treating parasites, especially sea lice, in fish.

Suitable additional active agents, that may be effective in treating parasitic sea lice and that may be used in a combination product together with diafenthiuron include additional parasiticides. The term "parasiticide" refers to any substance that is capable of depleting the parasite population, for example by killing or preventing growth or reproduction of the parasites, or otherwise causing the loss or removal of parasites from the host fish. The substance may target a broad range of parasites, or may be specific for a small group of parasites, such as an individual type of parasite. Preferably, the additional active agent is an agent that is effective for use in treating sea lice in fish.

The one or more additional parasiticide may be within the group of Acetylcholineesterase (AchE) inhibitors, GABA-gated chloride channel antagonists, GABA-gated chloride channel inhibitors, Sodium channel modulators, Nicotinic acetylcholine receptor (nAChR) agonists, Nicotinic acetylcholine receptor (aAChR) allosteric activators, chloride channel activators, juvenile hormone mimics, modulators of Chordontonal Organs, inhibitors of mitochondrial ATP synthase, uncouplers of oxidative phosphorylation via disruption of the proton gradient, Nicotinic acethylcholine receptor (nAChR) channel blockers, inhibitors of chitin biosynthesis, type 0 and type 1, moulting disruptor, ecdysone receptor agonists, octopamine receptor agonists, mitochondrial complex III electron transport inhibitors, Mitochondrial complex I electron transport inhibitors, Voltage-dependent sodium channel blockers, inhibitors of acetylCoA carboxylase, Mitochondrial complex II electron transport inhibitors, Ryanodine receptor modulators, tubulin binders, acethylcholineesterase (AChE) mimetics, uncouplers of the oxidative phosphorylation.

For example, the one or more additional parasiticide may be: a sodium channel modulator, which may be a pyrethroid, such as deltamethrin, or an oxadiazine, such as indoxacarb; a chloride channel modulator, which may be an avermectin, such as emamectin, or ivermectin, or may be a macrolide such as moxidectin, or a milbemycin such as milbemycin oxime; a neurotoxin, which may be a neonicotinoid, such as nitenpyram, or a triazine, such as cyromazin; a pyridine, such as pymetrozine; or a benzoylurea, which may be a chitin synthesis inhibitor. The chitin synthesis inhibitor which may be used in a combination product together with diafenthiuron may be, for example, bistrifluron, chlorfluazuron, flucycloxuron, flufenoxuron, hexaflumuron, novaluron, noviflumuron, buprofezin, diflubenzuron, fluazuron, lufenuron, and teflubenzuron. Preferred chitin synthesis inhibitors include diflubenzuron, lufenuron, and hexaflumuron.

The one or more additional active agent(s) that may be present in the combination formulation together with diafenthiuron may be present in the free form, or in any active form, such as in the form of any veterinarily acceptable salt.

A suitable combination treatment comprising diafenthiuron and an additional active agent, may be performed, for example, by treating the fish, in particular salmon, initially with said additional active agent, and thereafter, for example 1 week to 3 month or more, preferably 3 to 5 months or more, more preferably 6 months or more and in particular 8 to 12 months after the end of the treatment with said additional active agent, performing a treatment with diafenthiuron as described above. According to a preferred embodiment of this combination treatment, the first treatment is an in-feed treatment with hexaflumuron or in particular lufenuron, or with another active ingredient with long lasting protection against sea lice such as emamectin benzoate, which takes place at the end of the fresh water phase of salmon development or at the beginning of their sea water phase.

An alternative combination treatment comprising diafenthiuron and an additional active agent may be performed, for example, by treating the fish, in particular salmon, initially with diafenthiuron according to the in-feed method and regime as mentioned above, and thereafter, for example 3 months, preferably 5 months, more preferably 6 months and in particular 9 months following the end of the diafenthiuron in-feed treatment performing a treatment with the additional active agent; said second treatment may be a bath treatment, an in-feed treatment or preferably a treatment by injecting the additional active agent to the fish. According to a preferred embodiment of this combination treatment, the in-feed treatment with the diafenthiuron takes place at the end of the fresh water phase of salmon development or at the beginning of their sea water phase.

A further alternative combination treatment comprises first of all treating the fish, in particular salmon, with the additional active agent and thereafter, for example 1 hour to 2 months thereafter, preferably 1 hour to 1 month thereafter or in particular 1 week to 1 month thereafter, performing a diafenthiuron in-feed treatment as described above. According to a preferred embodiment of this combination treatment, the treatment with the additional active agent is a bath treatment, an in-feed treatment or injectable treatment which takes place at the beginning of the sea water phase, for example 1 hour to 3 months, preferably 6 hours to 2 months and in particular 12 hours to 1 month following the release of the fish to sea water.

### Pharmaceutical kits

The description also provides a pharmaceutical pack or kit comprising one or more containers filled with the formulation of the invention. Additionally provided in the kit is notice in the form prescribed by a governmental agency regulating the manufacture, use or sale of the pharmaceuticals, and an instruction for use of the pharmaceutical.

### Examples

The invention will now be explained in further detail in the following Examples, which demonstrate the discovery of a therapeutic window in which diafenthiuron may be used to effectively kill parasitic sea lice but is not toxic to the target fish.

Bioassays for *Lepeophtheirus salmonis* (Lesa assay) copepodites and pre-adults were used to test the potential of diafenthiuron for use in treating fish parasites, and in particular, sea lice.

### Example 1 - Effects on Sea Lice

The test diafenthiuron formulations were prepared by dissolving 250 mg diafenthiuron under stirring at ambient temperature in a premix of solvent (acetone, 1.5 ml) and solubilizer (Kolliphor EL, 1ml). The stock was then diluted in saltwater to the appropriate concentration.

Diafenthiuron concentrations of 20 ppb, 0.5 ppm and 2ppm were tested.

Control formulations were also prepared comprising only solvent (acetone) and solubilizer (Kolliphor EL) in the same ratio as the test formulation. A salt water control was also used.

Bioassay experiments were then conducted on copepodites and pre-adult/adult salmon lice (*Lepeophtheirus salmonis*)*.*

The bioassays involved exposing sea lice for 1 hour to seawater containing a particular concentration of diafenthiuron, or a control formulation. After 1 hour exposure, the lice were transferred to modified 6 well plates and washed twice in saltwater. The plates were then incubated and the numbers of dead and surviving lice were monitored until 96 hours post exposure. The experimental temperature was maintained at 8-10 °C.

The effect of different concentrations of diafenthiuron on the lice is shown in Table 1.

**Table 1**

| | **Stage** | **Dead/total** | **% dead** |
|---|---|---|---|
| Diafenthiuron 20 ppb | Copepodites | 11/23 | 48% |
| Diafenthiuron 0.5 ppm | Copepodites | 10/13 | 77% |
| Diafenthiuron 2.0 ppm | Copepodites | 20/20 | 100% |
| Placebo | Pre-adult/adult | 0/5 | 0 |
| Diafenthiuron 2.0 ppm | Pre-adult/adult | 5/5 | 100% |

The fact that diafenthiuron is capable of killing sea lice highly efficiently, and at different stages in the sea lice life cycle, is surprising and offers a significant advantage over many currently available treatments for parasitic sea lice.

### Example 2 - Toxicity to Fish (Bath Treatment)

Diafenthiuron has previously been reported to be toxic to fish. The effect of bath treatment of Atlantic salmon with diafenthiuron was tested.

In view of the fact that a diafenthiuron concentration of 0.5 ppm has been found to be toxic to sea lice (see above), concentrations greater than this level were tested for toxic effects on fish. Specifically, diafenthiuron concentrations of 2 ppm and 5 ppm in sea water were tested.

In the tests for diafenthiuron toxicity, six Atlantic salmon per group, having an average weight of 32.8 grams, were acclimatized to 12°C and were then placed in closed and oxygenated treatment containers and treated with the relevant concentration of diafenthiuron (2 ppm or 5 ppm), or a control formulation. The fish were kept in the tanks for 1 hour. After treatment the fish were removed from the treatment containers to observation tanks and observed for at least three days.

The effects on the fish of the diafenthiuron treatment were observed 1 hour post exposure and regularly until at least 72 hours post exposure. Neither concentration of diafenthiuron was found to induce any mortality or toxic effects on the fish.

### Example 3 - Toxicity to Fish (Intraperitoneal Injection)

The effect of injection treatment of tilapia (Oreochromis niloticus) with diafenthiuron was tested.

Diafenthiuron was formulated in a concentration of 25 mg/ml in PEG400 (85%)/DMSO (15%). An identical formulation without diafenthiuron was also prepared. Tilapia of mean weight of 158 grams reared in freshwater at 29°C were distributed in three groups of 10 fish. Each group was injected with 0.05 ml of the relevant formulation, or PBS control. The effects on the fish were observed for five days post treatment, and the results are shown in Table 2.

**Table 2**

| | **PBS** | **PEG400/DMSO** | |
|---|---|---|---|
| Diafenthiuron concentration (mg/ml) | 0 | 0 | 25 |
| No. of fish healthy after 5 days | 10/10 | 9/10 | 9/10 |
| % mortality | 0 | 10 | 10 |

Five days after treatment with diafenthiuron 90% of the treated fish were alive and healthy. Exactly the same proportion of fish was observed to be healthy in the group treated with the same formulation, but containing no diafenthiuron. This indicates that the low level of observed mortality was not due to diafenthiuron.

### Example 4 - Treatment of Sea Lice Infested Atlantic salmon

Diafenthiuron was tested for use in treating fish parasites. Specifically, the effect of bath treatment of sea lice infested Atlantic salmon (Salmo salar) was investigated.

Atlantic salmon were acclimatized to 12°C seawater. Following transfer to seawater, the fish were challenged with sea lice copepodids (L. salmonis). When attached lice had reached the adult stage, the number of attached female sea lice was counted.

The fish were then treated with 2ppm diafenthiuron for 60 minutes by bath. Infected fish treated with a formulation lacking diafenthiuron were used as a control group.

Three days after treatment the number of attached female sea lice was counted. The number of adult female sea lice was reduced by 56% in the treated group.

There was no toxic effect of diafenthiuron in the treated fish.

These results clearly demonstrate that there is a therapeutic window in which diafenthiuron can be used to effectively kill parasitic sea lice but has no toxic effect on the host fish.

Specifically, diafenthiuron may be used to treat a population of fish that contains parasitic sea lice. Specifically, it has surprisingly been found that it is possible to provide diafenthiuron for use in the treatment of fish parasites without any toxic effects on the fish. In this way, diafenthiuron may be used to treat parasitic sea lice.

## Claims

1. Diafenthiuron, or a veterinarily acceptable salt thereof, for use in treating parasitic sea lice infestations in fish.

2. Diafenthiuron, or a veterinarily acceptable salt thereof, for use as claimed in claim 1, wherein the sea lice are of the *Dissonus, Lepeophtheirus,* or *Caligus* genus of the Caligidae family, and said lice are copepodites or pre-adult sea lice.

3. Diafenthiuron, or a veterinarily acceptable salt thereof, for use as claimed in any one of claims 1 and 2, wherein the fish are of the Salmonidae or Cichlidae family.

4. Diafenthiuron, or a veterinarily acceptable salt thereof, for use as claimed in any one of claims 1-3, wherein a parasitically effective amount of the diafenthiuron or diafenthiuron salt is present in a veterinarily acceptable formulation.

5. Diafenthiuron, or a veterinarily acceptable salt thereof, for use as claimed in claim 4, wherein the formulation comprises a hydrophilic polymer or a non-aqueous polar solvent, or mixture thereof.

6. Diafenthiuron, or a veterinarily acceptable salt thereof, for use as claimed in claim 5, wherein the hydrophilic polymer comprises poly(ethylene glycol) or poly(propylene) glycol, or a derivative thereof.

7. Diafenthiuron, or a veterinarily acceptable salt thereof, for use as claimed in claim 5, wherein the non-aqueous polar solvent comprises one or a combination of DMSO, NMP, Tetraglycol, acetone, or DMF.

8. Diafenthiuron, or a veterinarily acceptable salt thereof, for use as claimed in any one of claims 4-7, wherein the formulation comprises a solubilizer.

9. Diafenthiuron, or a veterinarily acceptable salt thereof, for use as claimed in claim 8, wherein the solubilizer comprises a polyoxyethyene sorbitan fatty acid ester, polyoxyethylene castor oil derivative or a combination thereof.

10. Diafenthiuron, or a veterinarily acceptable salt thereof, for use as claimed in claims 4-9, wherein the formulation is suitable for dispersing the diafenthiuron or diafenthiuron salt in water containing the fish to a final diafenthiuron concentration of 0.05-5 ppm.

11. Diafenthiuron, or a veterinarily acceptable salt thereof, for use as claimed in claims 4-9, wherein the diafenthiuron or diafenthiuron salt is in the form of medicated fish feed.

12. Diafenthiuron, or a veterinarily acceptable salt thereof, for use as claimed in claim 11, wherein the medicated fish feed is in the form of fish feed granules or pellets which have a coating comprising the diafenthiuron or diafenthiuron salt.

13. Diafenthiuron, or a veterinarily acceptable salt thereof, for use as claimed in claims 4-9, wherein the diafenthiuron or diafenthiuron salt is formulated for intraperitoneal injection.

14. Diafenthiuron, or a veterinarily acceptable salt thereof, for use as claimed in claim 13, wherein the diafenthiuron or diafenthiuron salt is present in the formulation in an amount of 10-100 mg/ml.

15. Diafenthiuron, or a veterinarily acceptable salt thereof, for use as claimed in any one of claims 4-14, wherein the formulation comprises the diafenthiuron or diafenthiuron salt in combination with an additional active agent that is capable of treating fish against fish parasites.

## Patentansprüche

1. Diafenthiuron oder ein Veterinär verträgliches Salz davon zur Verwendung in der Behandlung von parasitären Seeläuse-Infestationen bei Fischen.

2. Diafenthiuron oder ein Veterinär verträgliches Salz davon zur Verwendung wie in Anspruch 1 beansprucht, wobei die Seeläuse zur Gattung *Dissonus, Lepeophtheirus* oder *Caligus* der Caligidae-Familie gehören und die Seeläuse Copepoditen oder prä-adulte Seeläuse sind.

3. Diafenthiuron oder ein Veterinär verträgliches Salz davon zur Verwendung wie in einem der Ansprüche 1 und 2 beansprucht, wobei die Fische zur Familie Salmonidae oder Cichlidae gehören.

4. Diafenthiuron oder ein Veterinär verträgliches Salz davon zur Verwendung wie in einem der Ansprüche 1-3 beansprucht, wobei eine parasitär wirksame Menge des Diafenthiuron oder Diafenthiuron-Salzes in einer Veterinär verträglichen Formulierung vorliegt.

5. Diafenthiuron oder ein Veterinär verträgliches Salz davon zur Verwendung wie in Anspruch 4 beansprucht, wobei die Formulierung ein hydrophiles Polymer oder ein nicht-wässriges polares Lösungsmittel oder ein Gemisch davon umfasst.

6. Diafenthiuron oder ein Veterinär verträgliches Salz davon zur Verwendung wie in Anspruch 5 beansprucht, wobei das hydrophile Polymer Poly(ethylenglycol) oder Poly(propylen)glycol oder ein Derivat davon umfasst.

7. Diafenthiuron oder ein Veterinär verträgliches Salz davon zur Verwendung wie in Anspruch 5 beansprucht, wobei das nichtwässrige polare Lösungsmittel eins oder eine Kombination von DMSO, NMP, Tetraglycol, Aceton oder DMF umfasst.

8. Diafenthiuron oder ein Veterinär verträgliches Salz davon zur Verwendung wie in einem der Ansprüche 4-7 beansprucht, wobei die Formulierung einen Solubilisator umfasst.

9. Diafenthiuron oder ein Veterinär verträgliches Salz davon zur Verwendung wie in Anspruch 8 beansprucht, wobei der Solubilisator einen Polyoxyethylensorbitanfettsäureester, ein Polyoxyethylen-Rizinusöl-Derivat oder eine Kombination davon umfasst.

10. Diafenthiuron oder ein Veterinär verträgliches Salz davon zur Verwendung wie in den Ansprüchen 4-9 beansprucht, wobei die Formulierung zum Dispergieren des Diafenthiuron oder des Diafenthiuron-Salzes in Wasser, das die Fische enthält, zu einer End-Diafenthiuron-Konzentration von 0,05-5 ppm geeignet ist.

11. Diafenthiuron oder ein Veterinär verträgliches Salz davon zur Verwendung wie in den Ansprüchen 4-9 beansprucht, wobei das Diafenthiuron oder das Diafenthiuron-Salz in der Form von Medizinalfischfuttermittel ist.

12. Diafenthiuron oder ein Veterinär verträgliches Salz davon zur Verwendung wie in Anspruch 11 beansprucht, wobei das Medizinalfischfuttermittel in der Form von Fischfuttermittel-Körnern oder -Pellets ist, die eine Beschichtung haben, die das Diafenthiuron oder das Diafenthiuron-Salz umfasst.

13. Diafenthiuron oder ein Veterinär verträgliches Salz davon zur Verwendung wie in den Ansprüchen 4-9 beansprucht, wobei das Diafenthiuron oder das Diafenthiuron-Salz zur intraperitonealen Injektion formuliert ist.

14. Diafenthiuron oder ein Veterinär verträgliches Salz davon zur Verwendung wie in Anspruch 13 beansprucht, wobei das Diafenthiuron oder das Diafenthiuron-Salz in einer Menge von 10-100 mg/ml in der Formulierung vorliegt.

15. Diafenthiuron oder ein Veterinär verträgliches Salz davon zur Verwendung wie in einem der Ansprüche 4-14 beansprucht, wobei die Formulierung das Diafenthiuron oder das Diafenthiuron-Salz in Kombination mit einem zusätzlichen Wirkstoff, der geeignet ist, Fische gegen Fischparasiten zu behandeln, umfasst.

## Revendications

1. Diafenthiuron, ou un sel de celui-ci acceptable vétérinaire, pour son utilisation dans le traitement des infestations parasitaires par les poux du poisson chez les poissons.

2. Diafenthiuron, ou un sel de celui-ci acceptable vétérinaire, pour son utilisation selon la revendication 1, dans lequel les poux du poisson sont du genre *Dissonus, Lepeophtheirus,* ou *Caligus* de la famille des *Caligidae,* et lesdits poux sont des copépodes ou des poux du poisson pré-adultes.

3. Diafenthiuron, ou un sel de celui-ci acceptable vétérinaire, pour son utilisation selon l'une des revendications 1 et 2, dans lequel les poissons sont de la famille des *Salmonidae* ou des *Cichlidae.*

4. Diafenthiuron, ou un sel de celui-ci acceptable vétérinaire, pour son utilisation selon les revendications 1 à 3, dans lequel une quantité efficace sur le plan parasitaire du diafenthiuron ou du sel de diafenthiuron est présente dans une formulation acceptable vétérinaire.

5. Diafenthiuron, ou un sel de celui-ci acceptable vétérinaire, pour son utilisation selon la revendication 4, dans lequel la formulation comprend un polymère hydrophile ou un solvant polaire non aqueux, ou un mélange de ceux-ci.

6. Diafenthiuron, ou un sel de celui-ci acceptable vétérinaire, pour son utilisation selon la revendication 5, dans lequel le polymère hydrophile comprend le poly(éthylène glycol) ou le poly(propylène)glycol, ou un dérivé de ceux-ci.

7. Diafenthiuron, ou un sel de celui-ci acceptable vétérinaire, pour son utilisation selon la revendication 5, dans lequel le solvant polaire non aqueux comprend un ou une combinaison de DMSO, de NMP, de tétraglycol, d'acétone ou de DMF.

8. Diafenthiuron, ou un sel de celui-ci acceptable vétérinaire, pour son utilisation selon l'une des revendications 4 à 7, dans lequel la formulation comprend un solubilisant.

9. Diafenthiuron, ou un sel de celui-ci acceptable vétérinaire, pour son utilisation selon la revendication 8, dans lequel le solubilisant comprend un ester d'acide gras de polyoxyéthylène sorbitane, un dérivé d'huile de ricin de polyoxyéthylène ou une combinaison de ceux-ci.

10. Diafenthiuron, ou un sel de celui-ci acceptable vétérinaire, pour son utilisation selon les revendications 4 à 9, dans lequel la formulation est adaptée pour la dispersion du diafenthiuron ou du sel de diafenthiuron dans l'eau contenant les poissons à une concentration de diafenthiuron finale de 0,05 à 5 ppm.

11. Diafenthiuron, ou un sel de celui-ci acceptable vétérinaire, pour son utilisation selon les revendications 4 à 9, dans lequel le diafenthiuron ou le sel de diafenthiuron est sous la forme d'un aliment pour poissons médicamenté.

12. Diafenthiuron, ou un sel de celui-ci acceptable vétérinaire, pour son utilisation selon la revendication 11, dans lequel l'aliment pour poissons médicamenté est sous la forme de granules ou comprimés alimentaires pour poissons qui présentent un enrobage comprenant le diafenthiuron ou le sel de diafenthiuron.

13. Diafenthiuron, ou un sel de celui-ci acceptable vétérinaire, pour son utilisation selon les revendications 4 à 9, dans lequel le diafenthiuron ou le sel de diafenthiuron est formulé pour une injection intrapéritonéale.

14. Diafenthiuron, ou un sel de celui-ci acceptable vétérinaire, pour son utilisation selon la revendication 13, dans lequel le diafenthiuron ou le sel de diafenthiuron est présent dans la formulation en une quantité de 10 à 100 mg/ml.

15. Diafenthiuron, ou un sel de celui-ci acceptable vétérinaire, pour son utilisation selon les revendications 4 à 14, dans lequel la formulation comprend le diafenthiuron ou le sel de diafenthiuron en combinaison avec un agent actif supplémentaire qui est capable de traiter les poissons contre les parasites du poisson.
